# EUROPEAN PATENT SPECIFICATION

(11) **EP 3 260 088 B1**
(45) Date of publication and mention of the grant of the patent: **01.07.2020**
(21) Application number: 16305766.4
(22) Date of filing: 24.06.2016
(51) Int. Cl.: A61F 2/46

(54) **SYSTEM FOR MONITORING BY-IMPACTION ASSEMBLY BETWEEN A PROSTHETIC COMPONENT AND A SUPPORT MEMBER, AND SURGICAL KITS INCLUDING SUCH A SYSTEM**
SYSTEM ZUR ÜBERWACHUNG EINER BY-IMPACTION-ANORDNUNG ZWISCHEN EINER PROTHESENKOMPONENTE UND EINEM STÜTZELEMENT UND CHIRURGISCHE KITS MIT SOLCH EINEM SYSTEM
SYSTÈME DE CONTRÔLE D'ENSEMBLE BY-IMPACTION ENTRE UN COMPOSANT DE PROTHÈSE ET UN ÉLÉMENT DE SUPPORT, ET TROUSSES CHIRURGICALES COMPRENANT UN TEL SYSTÈME

(43) Date of publication of application: 27.12.2017
(73) Proprietor: Tornier, 38330 Montbonnot-Saint-Martin (FR)
(72) Inventor: PIRIOU, Philippe, 95270 BELLOY EN FRANCE (FR); RENAULT, Eric, 38660 LUMBIN (FR); DERANSART, Pierric, 38410 SAINT MARTIN D'URIAGE (FR); CARDON, Jean-Emmanuel, 38420 DOMENE (FR)
(74) Representative: Lavoix

(56) References cited:
- EP-A2- 2 491 873
- WO-A1-2010/111272
- WO-A1-2015/187876
- DE-A1-102006 030 343
- US-A1- 2005 101 962
- US-A1- 2008 125 671

## Description

The present invention relates to a system for monitoring use of an impact application device operated by a user to assemble a prosthetic component with a support member by impaction, the impact application device including a hand hammer and an impactor intended to transmit force from the hammer to the prosthetic component. The invention also relates to a method for monitoring use of such an impact application device to assemble a first prosthetic component with a second prosthetic component by impaction.

In the field of human joint replacement surgery, surgeons often have to assemble a prosthetic component with a bone or with another prosthetic component. A common surgical technique is to impact the prosthetic component so as to block or jam a feature of the prosthetic component with a matched feature of the bone or of the other prosthetic component, in particular when no cement is used or when a thin cement layer is interposed between the aforesaid features. As the force to apply to the prosthetic component is high, the surgeon uses an impact application device to impact the prosthetic component so as to engage the aforesaid features with each other: the impact application device typically includes a hammer, which is manipulated by hand by the surgeon, and an impactor, which is a surgical tool designed to be interposed between the hammer and the prosthetic component so as to transmit force of the hit of the hammer thereon to the prosthetic component.

In practice, several impacts are necessary in order to fully assembly the prosthetic component with the bone or with the other prosthetic component and thus to be sure that the assembly is fully seated and therefor stable. A fully seated assembly is also expected in order to promote osseointegration when the prosthetic component is assembled with a bone. The prosthetic component is considered as being partially or not fully assembled with a bone or with another prosthetic component when in use, the prosthetic component is likely to move or even to disassemble with respect to the bone or the other prosthetic component. Such a partial or not fully seated assembly may occur for example when a gap remains between the aforesaid features or between at least respective parts of these features, which are intended to be in full contact. Another example of such a partial assembly is when assembly force between the aforesaid features is not sufficient even if no gap remains between them, which may typically the case for a Morse taper assembly between two prosthetic components.

However, the specific number of impacts that is necessary for the fully seated assembly is not predetermined and may depend on factors such as the force of each of the successive impacts, implant type, patient anatomy and the surgeon's ability. In order to be sure that the assembly is fully seated, the surgeons tend to strike the prosthetic component with the impact application device a number of times which is much higher than what is necessary. Thus, the total number of the impacts that can be given is twice or three times what is necessary. This excess of impacts may lead to weaken or even break the prosthetic component and/or the support member, i.e., bone or another component, with which the prosthetic component is assembled by impaction. For example, in the case of an acetabular cup to be assembled cementless and by-impaction into the acetabulum of the pelvis of a patient, continuing to strike the cup whereas this cup is already fully assembled into the acetabulum risks of fracturing the pelvis. According to another example, in the case of a prosthetic humeral head, especially in pyrocarbon, to be assembled cementless and by-impaction with a prosthetic humeral stem that is already or not yet implanted in the humerus of a patient, continuing to strike the head whereas this head is already fully assembled with the stem risks of damaging the head. Other examples can be considered for prosthetic components to be assembled with a support member, either osseous or prosthetic, by impaction and, if appropriate, without cement. In any case, the number of impaction impacts should be reduced at the minimum for having a fully seated assembly without damaging the prosthetic component and/or the support member.

In practice, the surgeons have difficulties to evaluate whether the prosthetic component is fully impacted. Some prosthetic components have a back side hole through which the surgeon can check the assembly, but using such a back side hole may be difficult in case of minimally invasive surgical techniques and is even not possible for numerous prosthetic components which are not provided with such a back side hole. Otherwise, the surgeon can ask intraoperative radiographic images, but that increases the operating time and radiation to the patient. Thus, the surgeon often stops to strike the prosthetic component only when he or she has the manual feeling that the assembly is fully seated. Of course, such a manual feeling is different between surgeons and is not the same depending on the prosthesis, the impact application device, and the bone quality.

This issue has already been considered in US 7 879 043. This document discloses a system and method for preventing intraoperative fracture in cementless hip arthroplasty. This system uses damage identification techniques based on vibration characteristics associated with femoral component impaction to determine when a femoral prosthetic stem is fully assembled into the femur of a patient. More precisely, the system comprises accelerometers that are attached to a large neck of the femoral stem: each time a hammer impacts the upper end of the femoral stem, the femoral stem is moved into the femur and the accelerometers provide a signal representative of acceleration of the femoral stem. A temporal analysis of the acceleration signals, which are provided for successive impacts, enables to warn the surgeon that the full assembly is reached. In practice, this system is quite difficult to use because the accelerometers thereof need to be attached to the femoral stem: the accelerometers need to be sterilized before being placed on the femoral stem in the sterile surgical field and they risk being damaged by the hammer. Besides, unlike a femoral stem that includes a large neck, numerous prosthetic components cannot be equipped with such attached accelerometers.

Another approach is proposed in DE 10 2006 030 343 which discloses a system for controlling a motorized, impact application device held by a surgeon to assemble a prosthesis with a bone. The impact application device incorporates a motorized percussion tool which is applied to a tool chuck firmly secured to the prosthesis. The system comprises a control unit that controls the impact application device on the basis of force detected by a force sensor and of parameters of a predetermined software. The system may further comprise a sound sensor, signal of which is also provided to the control unit to be compared to a sound pattern that is stored as a reference in a database of the control unit. The control unit automatically controls the motorized percussion tool, without interaction with the surgeon.

One of the goals of the present invention is to propose a system for monitoring impaction of a prosthetic component, which is easy and convenient to use, for any type of prosthetic component.

To this end, one object of the invention is a system as defined in appended claim 1. Other objects of the invention are surgical kits as respectively defined in the appended claims 12 and 14. Additional advantageous features of this system and of these kits are specified in dependent claims 2 to 11 and 13.

Another object of the invention is a method as defined in claim 15. This method is implemented by using the system as defined above. This method is carried out whereas the second prosthetic component is not yet implanted in a bone.

Another object disclosed therein is a method for monitoring by-impaction assembly between a prosthetic component and a bone, comprising:
- each time an impact application device applies an impact to the prosthetic component during assembly between the prosthetic component and the bone, producing data representing acoustic vibrations generated in the air and/or material vibrations generated in the impact application device, from at least one vibrational sensor,
- calculating a frequency characterization of the vibrations for each impact applied by the impact application device to the prosthetic component, from the corresponding data produced by the at least one vibrational sensor,
- comparing the frequency characterizations that are respectively calculated for successive impacts so as to provide at each of the successive impacts a first indication when the assembly between the prosthetic component and the bone is not fully seated then a second indication when the assembly between the prosthetic component and the bone is fully seated, and
- providing feedback to a user based on the first and second indications.

One of the ideas underlying the invention is to exploit not the vibrations generated in the prosthetic component each time the latter is hit by an impact application device in order to be assembled with the support member, but the vibrations that are generated in the air and/or in the impact application device. For this purpose, one or more vibrational sensors are used to detect the acoustic vibrations in the air and/or the material vibrations in the impact application device. In practice, this or these vibration sensors may not necessarily be attached to the prosthetic component. Thus, the invention can be used for any type of prosthetic components and simplifies or even avoids sterilization issue of these vibrational sensors.

Insofar as the data provided by the vibrational sensors are not directly representative of the displacement of the prosthetic component during the assembly between the prosthetic component and the support member, the invention proposes to analyze the frequency of the vibrations detected by the vibrational sensors: by calculating a frequency characterization of the vibrations generated for each impact applied by the impact application device and by comparing the respective frequency characterizations of the successive impacts of the impact application device, the invention enables to provide relevant information about the degree of impaction of the prosthetic component. In practice, various types of frequency characterization can be considered, such as a natural frequency for the acoustic vibrations in the air or a wave frequency for the material vibrations in the impact application device. Similarly, the type of feedback provided to the surgeon is not limitative for the invention, sound or light feedback being possible. In any case, as soon as an appropriate indication is given by the system of the invention utilizing methods described herein, and/or by the method of the invention, the surgeon can stop to strike the prosthetic component, while being sure that the assembly between the prosthetic component and the support member is fully seated and thus stable.

Embodiments of the invention will be better understood from reading the description which will follow, which is given solely by way of example and with reference to the drawings in which:
- figure 1 is a schematic view of a system for monitoring assembly between a hip prosthetic component and a pelvis;
- figures 2 and 3 are views similar to figure 1, which respectfully illustrate a partial assembly of the hip component and a fully seated assembly of the hip component;
- figure 4 is a diagram of a temporal acoustic signal produced during assembly of the hip component;
- figure 5 is a diagram of frequency characterizations calculated from the temporal signal of figure 4;
- figures 6, 7 and 8 are schematic views of various exemplary embodiments for the monitoring system of figure 1;
- figure 9 is a perspective view of humeral prosthetic components;
- figures 10 and 11 are schematic views of another monitoring system, which illustrate the use of this system for monitoring assembly between a first humeral component amongst those of figure 9 and a humerus, this assembly being partial in figure 10 and being fully seated in figure 11; and
- figures 12 and 13 are schematic views of another monitoring system, which illustrate the use of this system for monitoring assembly between the second and third humeral components of figure 9 and the first humeral component.

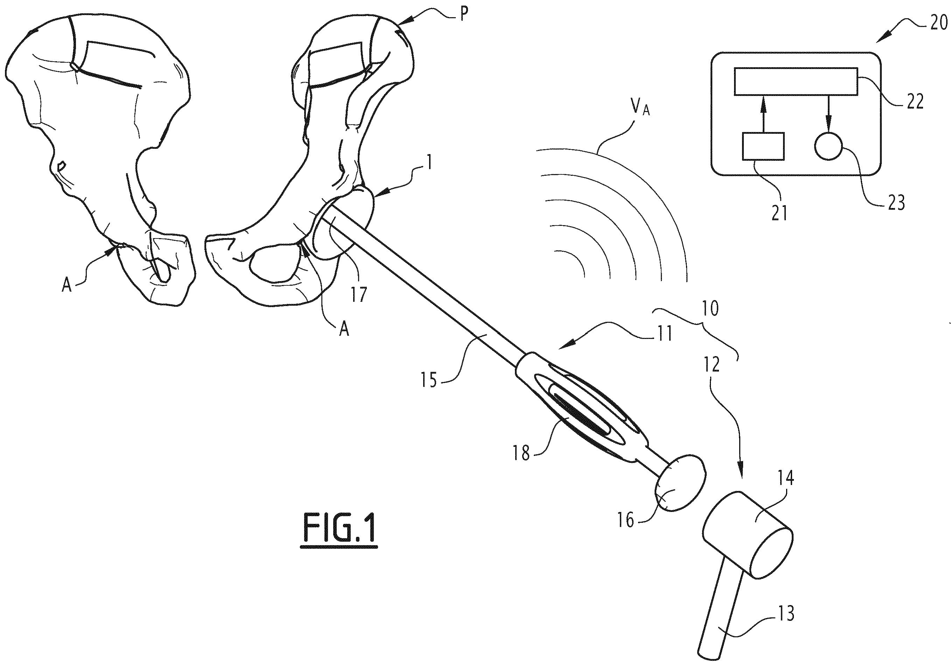

Figure 1 shows a human pelvis P having two acetabula A. Each of the acetabula is a cavity in the pelvis P, which articulates with the head of a (not shown) femur. Total hip arthroplasty replaces the joint between the pelvis and the femur with prosthetic components of a hip prosthesis, which articulates with each one other for replicating the natural hip joint and which are respectively assembled with the pelvis P and the femur. These prosthetic components typically include an acetabular cup 1. During surgery, the cup 1 is assembled with the pelvis P, within one of the acetabula thereof. As depicted on figure 1, the assembly of the cup 1 into the acetabulum A of the pelvis P is performed by impaction by using an impact application device 10 detailed here-after. This assembly is usually achieved without cement, that is to say without interposing surgical cement between the cup 1 and the acetabulum A. The cup 1 is known per se and is for example DYNACUP sold by TORNIER, France.

In some embodiments as the one shown in figure 1, the impaction application device 10 comprises an impactor 11 and a hammer 12. The hammer 12 is manipulated by hand by a surgeon and is consequently provided with a handle 13. The hammer 12 is also provided with a head 14 arranged at an end of the handle 13. The head 14 is designed to hit the impactor 11 and thus to apply an impaction force thereto from the surgeon. The impactor 11 is a surgical tool which is mainly intended to transmit impaction force from the hammer 12 to the acetabular cup 1. In some embodiments as the one shown in figure 1, the impactor 11 comprises a rod 15 having a proximal end 16 designed to be hit by the head 14 of the hammer 12 whereas a distal end 17 of the rod 15 is coupled to the cup 1.

The type of the coupling between the distal end 17 of the impactor 11 and the acetabular cup 1 is not limitative for the invention: in any case, this coupling is provided for driving the cup 1 into the acetabulum A by the rod 15 when the impactor 11 is hit by the hammer 12. In summary, the impaction application device allows the surgeon to apply an impact to the acetabular cup 1 in order to assemble this acetabular cup with the pelvis P.

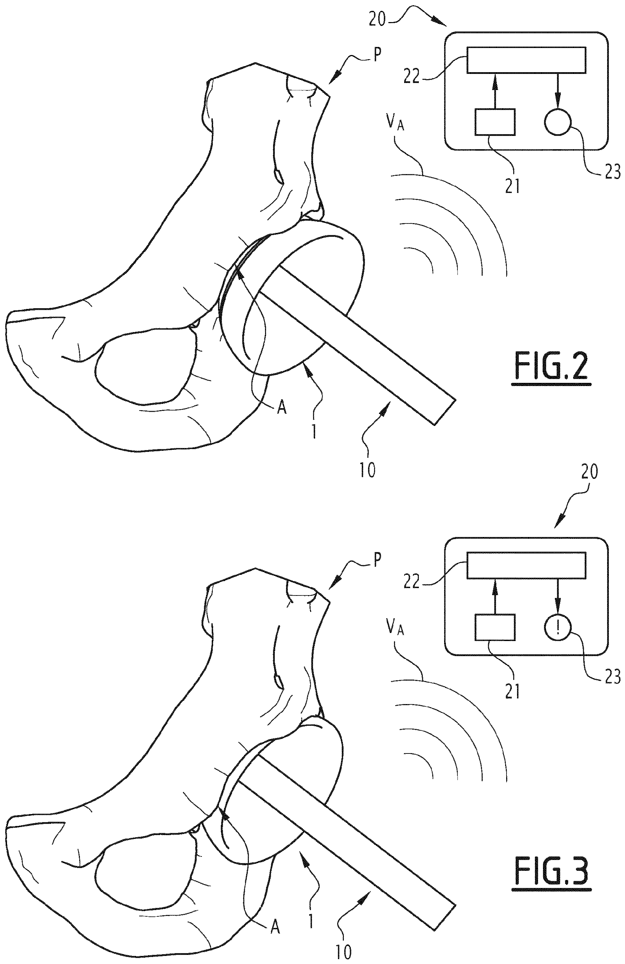

In practice, as explained in the introductive part of the present document, several impacts or hits are necessary so that the assembly between the acetabular cup 1 and the pelvis P is fully seated, or in other words full or complete or completely seated or properly positioned and assembled. Figure 2 depicts a not fully seated, or partial assembly, in which the acetabular cup 1 is not sufficiently engaged into the acetabulum A, whereas figure 3 depicts the assembly when it is fully seated or in other words completely assembled. Passing the assembly from the partial state, or in other words partially seated state, of figure 2 to the full state, or in other words fully seated state, of figure 3 requires applying several impacts to the acetabular cup by the impact application device 10 operated by the surgeon.

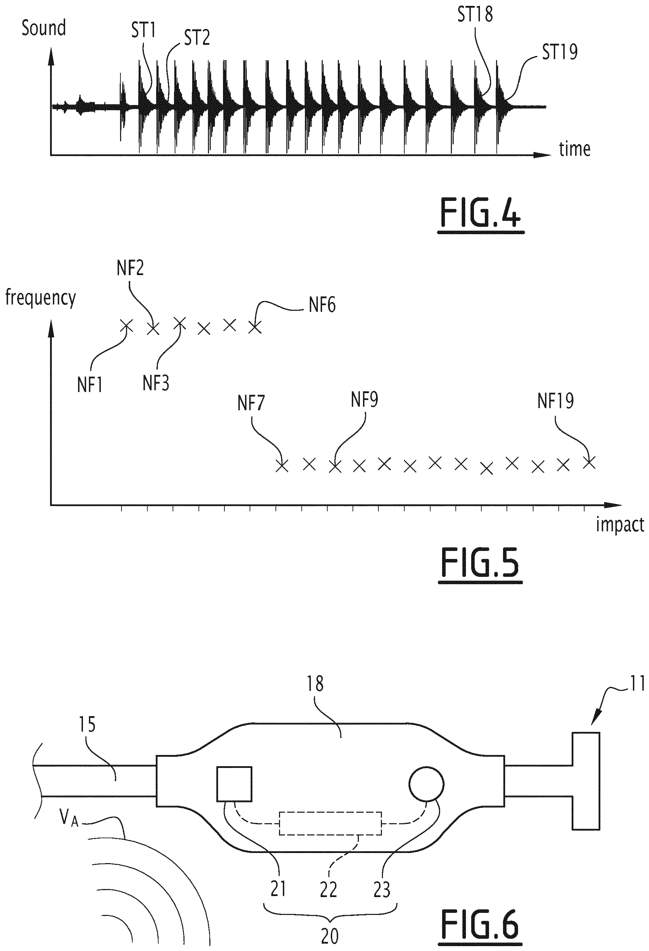

Referring back to figure 1, a monitoring system 20 is provided to allow the surgeon to monitor the assembly between the acetabular cup 1 and the pelvis P. The monitoring system 20 comprises a microphone 21, that is to say a vibrational sensor which both measures acoustic vibrations and produces data, typically an electronic signal, representing these acoustic vibrations. The microphone 21 is designed to be sensitive to the acoustic vibrations V_{A} which are generated in the air when an impact is applied by the impact application device 10 to the acetabular cup 1. In practice, microphone 21 is chosen to be sensitive to these acoustic vibrations V_{A} or is set to be sensitive to such acoustic vibrations. In some embodiments, the data which are produced by the microphone 21 are an acoustic signal as the one shown for example in figure 4. The signal of figure 4 corresponds to the sounds which are produced during surgery when successive impacts are applied to the cup 1 by the impact application device 10: in this specific and not limitative example, this temporal acoustic signal includes nineteen sound traces ST1, ST2,..., ST19, which are temporally successive and which respectively correspond to nineteen successive impacts applied to the cup 1. The time between two successive sound traces is not constant amongst the sound traces ST1 to ST19 because this time depends on the speed with which the surgeon manipulates the hammer 12 to hit the impactor 11. In the same way, the sound intensity of each of the sound traces is not necessarily constant amongst the sound traces ST1 to ST19 because the surgeon does not hit exactly with the same force.

The monitoring system 20 further includes an analysis unit 22 which is in communication with the microphone 21 and which receives the data produced by the microphone 21. The analysis unit 22 is configured to calculate a frequency characterization of the acoustic vibrations V_{A} for each impact applied by the impact application device 10 to the acetabular cup 1, from the corresponding data provided by the microphone 21. For this purpose, the analysis unit includes an appropriate calculator, for example a microprocessor or an electronic device which accepts as input the data coming from the microphone 21. In some embodiments, calculation performed by the analysis unit 22 corresponds to an FFT (fast Fourier transform) spectrum analysis which leads to calculate a natural frequency, as frequency characterization, for the acoustic vibrations V_{A} resulting from each impact. For example, figure 5 shows the result of such an FFT spectrum analysis for the temporal acoustic signal of figure 4: the natural frequency, also called fundamental frequency, of each of the nineteen sound traces ST1 to ST19 of figure 4 is calculated by the analysis unit 22, each of the calculated natural frequencies being represented as a point NF1, NF2,..., NF19 in figure 5. In this figure 5, the nineteen impacts, of which the respective acoustic vibrations V_{A} are measured by the microphone 21, are successively indicated along the abscissa of the diagram of figure 5, whereas the natural frequency associated to each of these nineteen impacts, as calculated by the analysis unit 22, is plotted on the ordinate of this diagram.

According to optional aspects, the analysis unit 22 can filter out the data coming from the microphone 21 before calculating the respective frequency characterizations of the acoustic vibrations V_{A} of the impacts. For example, the analysis unit 22 can include a background noise filter and/or a bandpass filter.

The analysis unit 22 is also configured to compare the frequency characterizations that are respectively calculated for the successive impacts applied to the acetabular cup 1 by the impact application device 10, so as to provide at each of these successive impacts either a first indication when the assembly between the acetabular cup 1 and the pelvis P is not full or a second indication when the assembly between the cup 1 and the pelvis P is fully seated. This comparison implemented by the analysis unit 12 is intended to indicate a substantial difference between the successive calculated frequency characterizations.

In some embodiments, this comparison is made, at each of the successive impacts, between the frequency characterization of the acoustic vibrations V_{A} measured by the microphone 21 at the last impact with the frequency characterization of the acoustic vibrations V_{A} measured by the microphone 21 at the second last impact: in the case in which the frequency characterizations are the natural frequencies of the acoustic vibrations as illustrated by figure 5, this comparison involves comparing, at the nth impact of the nineteen successive impacts, the natural frequency NFn with the natural frequency NF(n-1).

In some embodiments, rather to be the frequency characterization resulting from the last impact, the first member of the comparison implemented by the analysis unit 22 is an average of the frequency characterizations respectively resulting from the N last impacts, N being a number between two and ten, preferably between two and eight, preferably between two and six, and preferably between two and four. In some embodiments, rather to be the frequency characterization resulting from the second last impact, the second member of the comparison implemented by the analysis unit 22 is an average of the frequency characterizations respectively resulting from the M impacts just preceding the last impact, M being a number between two and ten, preferably two and eight, preferably two and six, and preferably two and four. Using one and/or the other of these averages in the comparison implemented by the analysis unit 22 can avoid distorting the result, for example due to one incorrect measurement or processing.

Also in some embodiments, rather to be the frequency characterization resulting from the second last impact or an average of the impacts just preceding the last impact, the second member of the comparison implemented by the analysis unit 22 is a threshold: this threshold can be predetermined, for example by calibration, or can be calculated, for example as a predetermined percent of the frequency characterization resulting from the first impact or an average of the frequency characterizations resulting from the several first impacts, for example the four first impacts, preferably the three first impacts and preferably the two first impacts.

Whatever the two members of the comparison implemented by the analysis unit 22, as long as, at each new impact amongst the successive impacts, these two members remain substantially equal to each other, for example equal to each other to about 25% or about 20% or about 15% or about 10% or about 5%, it means that the respective frequency characterizations resulting from the successive impacts applied so far are representative of the partial assembly between the acetabular cup and the pelvis P. However, as soon as, for an additional impact, the two compared members are considered by the analysis unit 22 as being different, it means that the assembly between the cup 1 and the pelvis P is now in its fully seated state and no more successive impacts are required. More generally, as long as the result of comparison implemented by the analysis unit corresponds to an unchanged value, to about 25% or about 20% or about 15% or about 10% or about 5%, for the frequency characterizations respectively associated to the impacts applied so far, the analysis unit 22 provides the first indication, that is to say the indication according to which the assembly between the cup 1 and the pelvis P is not fully seated; however, as soon as an additional impact implies a modification for the results of this comparison, the analysis unit 22 provides the second indication, that is to say the indication according to which the assembly between the cup 1 and the pelvis P is fully seated and therefor no additional impacts are necessary to fully seat the cup 1 with the pelvis P .

An illustration of the forgoing can be considered referring back to figure 5: in the case illustrated by figure 5, the six first natural frequencies NF1 to NF6 have approximately the same value whereas the seventh natural frequency NF7 and the following natural frequencies NF8 to NF19 have approximately the same value which is substantially lower than the value of the first six natural frequencies NF1 to NF6, which means that during surgery, the acetabular cup 1 is not fully assembled with the pelvis P after the six first impacts (corresponding to NF1-NF6) but the cup is fully assembled at the seventh impact (corresponding to NF7) and remains fully assembled for the following impacts (corresponding to NF8-NF19). The example of figure 5 also helps to understand that in the context of this example, the analysis unit 22 provides the first indication at least for the six first impacts and provides the second indication at the seventh impact or at an impact that closely follows the seventh impact depending on the specificities of the comparison implemented by the analysis unit. More generally, whatever the type of the comparison implemented by the analysis unit 22, this unit provides the second indication either at the "just enough" impact, that is to say the impact that ends up moving the cup 1 into the fully seated assembly with the pelvis P, or at an impact that closely follows the "just enough" impact, depending on the specificities of the comparison implemented by the analysis unit. Applying one or a few additional impacts beyond the "just enough" impact may confirm stabilization or the fully seated assembly.

In practice, calculation and comparison, which are implemented by the analysis unit 22, are processed in real-time and result from appropriate instructions which are stored in the analysis unit 22 or stored on a medium readable by this analysis unit.

Referring back to figure 1, the monitoring system 20 further comprises a user interface 23. The user interface 23 is in communication with the analysis unit 22 and receives from the analysis unit 22 a signal representative of the first and second indications which are provided by the analysis unit. The user interface 23 is designed to provide feedback based on these first and second indications: when the user interface 23 receives the first indication from the analysis unit 22, the user interface provides a first feedback element whereas when the user interface 23 receives the second indication, the user interface provides a second feedback element that is different from the first feedback element. In practice, the types of feedback provided by the user interface are not limitative so long as the various types of feedback are communicated to and understood by the surgeon. In some embodiments, feedback provided by the user interface 23 is visual: for example, the user interface 23 changes its color or its pattern. In this context, as illustrated by the figures 2 and 3, it can be considered that the user interface 23 remains blank when receiving the first indication from the analysis unit whereas the user interface 23 displays an exclamation mark "!" when receiving the second indication. In some embodiments, in addition to being visual or instead of being visual, the feedback provided by the user interface may be audible. Whatever the type of feedback provided by the user interface 23, this feedback gives the surgeon the information that the assembly between the acetabular cup 1 and the pelvis P is first partially seated, and then fully seated.

In use, the monitoring system 20 allows the surgeon to monitor the assembly between the acetabular cup 1 and the pelvis P. Indeed, each time the surgeon uses the impact application device 10 to apply an impact to the cup 1 in view of assembly thereof with the pelvis, the microphone 21 measures the acoustic vibrations V_{A} generated in the air by the impact and produces data representing these acoustic vibrations V_{A}. For each impact, these data are processed by the analysis unit 22 in real-time so as to calculate a frequency characterization of the corresponding acoustic vibrations V_{A}. By comparing the frequency characterizations that are respectively calculated for the successive impacts, the analysis unit 22 provides at each of the successive impacts the first indication then the second indication. As long as the surgeon has feedback from the user interface 23, which corresponds to the first indication, the surgeon continues to apply new impacts, but when the surgeon has feedback corresponding to the second indication, the surgeon can stop to apply any new impact while having the assurance that the assembly between the cup 1 and the pelvis P is fully seated.

Thus, unnecessary additional impacts are avoided. It can be noted that in the example given in the figures 4 and 5, the 8^{th} impact and the following impacts are unnecessary so that all of them or at least a majority of them could have been avoided if the surgeon had reacted to the corresponding feedback (e.g., stopping impaction or impact hits), shown in figure 3, given by the monitoring system 20.

As the monitoring system 20 exploits the acoustic vibrations V_{A} in the air, this system, especially the microphone 21 thereof, can be separate from the acetabular cup 1, that is to say can be not attached completely and rigidly to the cup 1, insofar as the data provided by the microphone 21 are not directly representative of the displacement of the cup 1 during the assembly thereof.

In some embodiments as the one shown in figure 6, the monitoring system 20 is integrated into the impactor 11 of the impact application device 10: in the example of figure 6, the microphone 21, the analysis unit 22 and the user interface 23 are integrated into the grip 18 of the impactor 11. A not shown variant is to integrate the monitoring system 20 into the hammer 12 of the impact application device 10. In both cases, sterilization requirements are less than for the acetabular cup 1 or for the distal end 17 of the impactor 14, which is coupled with the cup 1.

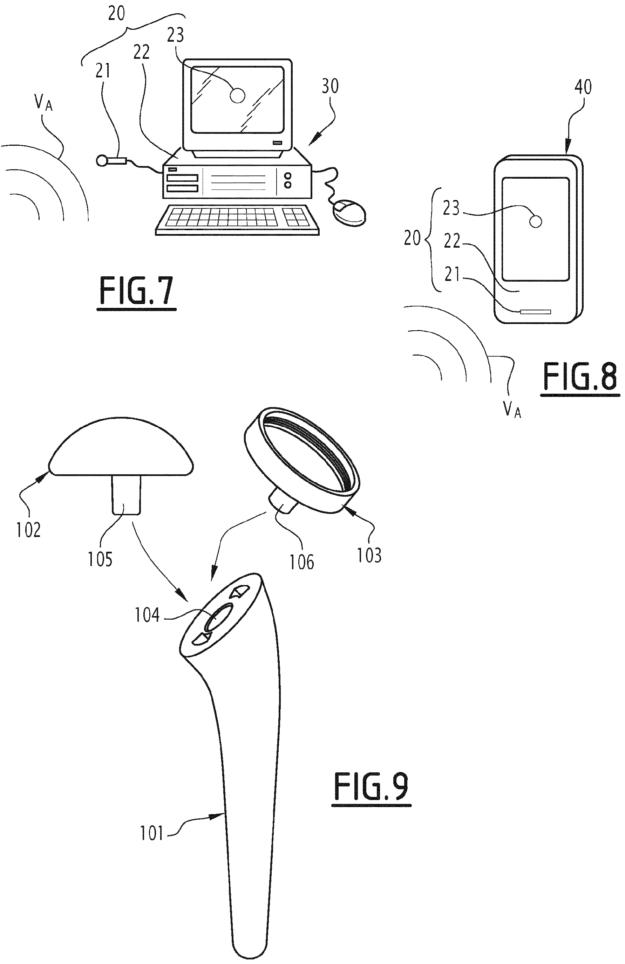

In some embodiments as the ones shown in the figures 7 and 8, the monitoring system 20 is also separate from the impact application device 10. In that case, the monitoring system 20 can be placed outside the sterile field of surgery. As depicted on figure 7, the monitoring system 20 can be integrated into a personal computer 30: the central unit of the computer 30 incorporates the analysis unit 22, the microphone 21 is formed by an appropriate peripheral of the computer 30, and at least a part of the screen of the computer 30 forms the user interface 23. As depicted on figure 8, the monitoring system 20 can be incorporated into a smartphone 40 as an example of any portable device that can integrate the monitoring system. Other examples of such portable devices are a laptop, a pad, etc. In the case of the smartphone 40, the microphone, the microprocessor and the screen of this smartphone respectively form or incorporate the microphone 21, the analysis unit 22 and the user interface 23 of the monitoring system 20.

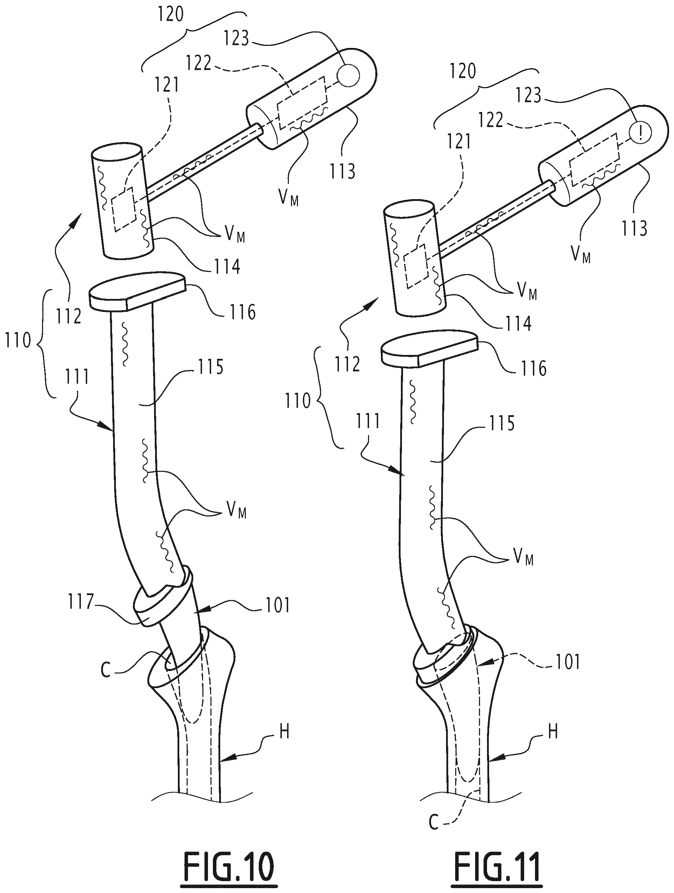

Figure 9 shows three humeral prosthetic components of a shoulder prosthesis which are respectively a humeral stem 101, a humeral head 102 and a humeral plate 103. As known per se and as depicted on the lower part of the figures 10 and 11, the humeral stem 101 is provided to be assembled with a human humerus H, by being introduced into a medullary canal C of the humerus H and assembled with this canal by impaction and with or without cement. The humeral head 102 and the humeral plate 103 are designed to be assembled with the humeral stem 101 by impaction: for example, as known per se and as shown in figure 9, the stem 101 is provided with an upper female feature 104 of a Morse connection, and the humeral head 102 and the plate 103 are each provided with a lower male feature 105, 106 of the Morse connection. The components 101, 102 and 103 are for example part of AEQUALIS shoulder prosthesis, sold by TORNIER, France. In some embodiments, the humeral head can be at least partly made of pyrocarbon.

Turning to the figures 10 and 11, an impact application device 110 and a monitoring system 120 will now be discussed in connection with the humeral stem 101.

The impact application device 110 allows the surgeon to apply successive impacts to the humeral stem 101 so as to assemble this humeral stem with the humerus H and thus move the corresponding assembly from a partial state (or partially seated), as shown in figure 10, to a full state (or fully seated), as shown in figure 11. For this purpose, the impact application device 110 includes an impactor 111 and a hammer 112. As explained above for the hammer 12 of the impact application device 10, the hammer 112 comprises a handle 113 and a head 114. As also explained above for the impactor 11 of the impact application device 10, the impactor 111 comprises a rod 115 having a proximal end 116, on which the head 114 of the hammer 112 can be hit, and a distal end 117, which is, in use, coupled to the humeral stem 101 so as to drive this stem at each hit of the hammer 112 on the impactor 111.

The monitoring system 120 allows the surgeon to monitor the assembly between the humeral stem 101 and the humerus H. Like the monitoring system 20, the monitoring system 120 comprises a vibrational sensor 121, an analysis unit 122 and a user interface 123.

Unlike the vibrational sensor 21 that is sensitive to acoustic vibrations in the air, the vibrational sensor 121 is sensitive to material vibrations V_{M} that are generated in the impact application device 110 at each impact applied by the impact application device 110 to the humeral stem 101. The sensor 121, which is a piezoelectric sensor in some embodiments, is integrated into the impact application device 110 and is operable to produce data representing the material vibrations V_{M}. As depicted on the figures 10 and 11, such a piezoelectric sensor is for example integrated into the head 114 of the hammer 112.

The analysis unit 122 is similar to the analysis unit 22, except that the analysis unit is configured to process the data coming from the sensor 121. In some embodiments, the frequency characterization, that is calculated by the analysis unit 122 for each impact applied by the impact application device 110 to the humeral stem 101, is a wave frequency of the corresponding material vibrations V_{M} generated in the impact application device.

The interface user 123 is similar or even identical to the interface user 23 of the monitoring system 20.

In some embodiments as the one shown in the figures 10 and 11, the analysis unit 122 and the user interface 123 are integrated in the hammer 112, for example in the handle 113 of the hammer.

In use of the monitoring system 120, each time the surgeon uses the impact application device 110 to apply an impact to the stem 101 in view of assembly thereof with the humerus H, the sensor 121 measures the material vibrations V_{M} generated in the impact application device 110 by the impact and produces data representing these material vibrations V_{M}. For each impact, these data are processed by the analysis unit 122 in real-time so as to calculate a frequency characterization of the corresponding material vibrations. By comparing the frequency characterizations that are respectively calculated for the successive impacts, the analysis unit 122 provides at each of the successive impacts the first indication then the second indication. As long as the surgeon has feedback from the user interface 123, which corresponds to the first indication, the surgeon continues to apply new impacts, but when the surgeon has feedback corresponding to the second indication, the surgeon can stop to apply any new impact while having the assurance that the assembly between the stem 101 and the humerus H is fully seated or complete.

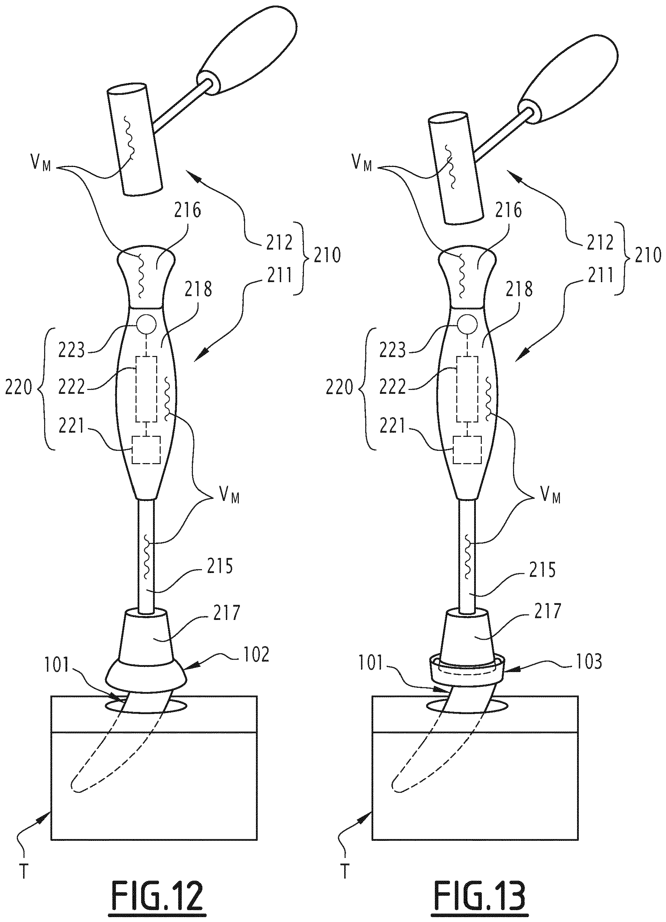

Turning to the figures 12 and 13, an impact application device 210 and a monitoring system 220 will now be briefly discussed in connection with the humeral head 102 and the humeral plate 103.

The impact application device 210 allows the surgeon to apply successive impacts selectively to the humeral head 102 and to the humeral plate 103 so as to assemble selectively this humeral head or this humeral plate with the humeral stem 101 and thus move the corresponding assembly, especially by the aforesaid Morse connection between the features 104 and 105 or between the features 104 and 106, from a partial state to a full state. Similarly to what has been explained above for the impact application devices 10 and 110, the impact application devices 210 includes an impactor 211 and a hammer 212. The impactor 211 comprises a rod 215 having a proximal end 216, on which can be hit the hammer 212, and a distal end 117, which is, in use, coupled selectively to the humeral head 102 or the humeral plate 103, the shape of this distal end 217 being slightly modified depending on the humeral component between the head 102 and the plate 103 to which it is coupled.

The monitoring system 220 is similar to the monitoring system 120 and comprises a vibrational sensor 221, an analysis unit 222 and a user interface 223 which are respectively similar to the sensor 121, the unit 122 and the interface 123.

In some embodiments as the one shown in the figures 12 and 13, the monitoring system 220 is integrated into the impactor 211, especially into a grip 218 of this impactor.

In use of the monitoring system 220, each time the surgeon uses the impact application device 210 to apply an impact to the head 102 in view of assembly thereof with the stem 101, the sensor 221 measures the material vibrations V_{M} generated in the impact application device 210 by the impact and produces data representing these material vibrations V_{M}. For each impact, these data are processed by the analysis unit 222 in real-time so as to calculate a frequency characterization of the corresponding material vibrations. By comparing the frequency characterizations that are respectively calculated for the successive impacts, the analysis unit 222 provides at each of the successive impacts the first indication then the second indication. As long as the surgeon has feedback from the user interface 223, which corresponds to the first indication, the surgeon continues to apply new impacts, but when the surgeon has feedback corresponding to the second indication, the surgeon can stop to apply any new impact while having the assurance that the assembly between the head 102 and the stem 101 is fully seated or complete. Same considerations apply when the monitoring system 220 is used during assembly between the humeral plate 103 and the stem 101. In both cases, during the assemblies, the stem 101 can be held in an impaction table T, known per se.

The various embodiments and examples given so far show that the invention can be used to monitor by-impaction assembly between any type of prosthetic components and a support member that is either a bone or another prosthetic component. Thus, in addition to the examples already given above, the invention can be used to monitor by-impaction assembly between a glenoid baseplate and glenoid bone of a human scapula, or between a femoral hip stem and medullary canal of a human femur. In addition to the examples already given above, the invention can also be used to monitor by-impaction assembly between a prosthetic insert and a prosthetic stem, or between a glenosphere and a glenoid baseplate, or between an acetabular ceramic insert and an acetabular cup, or between a humeral head, potentially comprising pyrocarbon, and a humeral anchorage.

Each feature of the monitoring systems 20, 120 and 220 may be implemented for any of the above-disclosed embodiments, when technically possible.

## Claims

1. System (20; 120; 220) for monitoring use of an impact application device (10; 110; 210) operated by a user to assemble a prosthetic component (1; 101; 102; 103) with a support member (P; H; 101) by impaction, the impact application device including a hand hammer (12; 112; 212) and an impactor (11; 111; 211) intended to transmit force from the hammer to the prosthetic component, the system comprising:
- at least one vibrational sensor (21; 121; 221) which is operable to produce data each time the impactor (11; 111; 211) is hit by the hammer (12; 112; 212) manipulated by hand by the user and thus applies an impact to the prosthetic component during assembly of the prosthetic component and the support member, the produced data representing acoustic vibrations (V_{A}) generated in the air and/or material vibrations (V_{M}) generated in the impact application device,
- an analysis unit (22; 122; 222) which is configured both to calculate a frequency characterization of the vibrations for each impact applied by the impact application device to the prosthetic component, from the corresponding data produced by the at least one vibrational sensor, and to compare the frequency characterizations that are respectively calculated for successive impacts so as to provide at each of the successive impacts either a first indication when the assembly between the prosthetic component and the support member is not fully seated or a second indication when the assembly between the prosthetic component and the support member is fully seated, and
- a user interface (23; 123; 223) which provides the user with feedback based on the first and second indications.

2. System according to claim 1, wherein the at least one vibrational sensor includes a microphone (21) operable to produce data representing the acoustic vibrations (V_{A}) generated in the air, the microphone being separate from the prosthetic component (1).

3. System according to claim 2, wherein the microphone (21) is also separate from the impact application device (10) .

4. System according to claim 3, wherein the microphone (21), the analysis unit (22) and the user interface (23) are integrated into a portable device (40), such as a laptop, a smartphone or a pad.

5. System according to claim 2, wherein the microphone (21) is integrated into the impact application device (10) .

6. System according to any one of the preceding claims, wherein the at least one vibrational sensor includes a piezoelectric sensor (121; 221) which is operable to produce data representing the material vibrations (V_{M}) generated in the impact application device (110; 210), the piezoelectric sensor being integrated into the impact application device.

7. System according to claim 5 or claim 6, wherein the analysis unit (22; 122; 222) and the user interface (23; 123; 223) are also integrated into the impact application device (10; 110; 210).

8. System according to any one of the preceding claims, wherein the frequency characterization calculated by the analysis unit (22) is a natural frequency (NF1 - NF19) of the acoustic vibrations (V_{A}) generated in the air.

9. System according to any one of the preceding claims, wherein the frequency characterization calculated by the analysis unit (122; 222) is a wave frequency of the material vibrations (V_{M}) generated in the impact application device (110; 210).

10. System according to any one of the preceding claims, wherein at each of the successive impacts, the analysis unit (22; 122; 222) is configured to compare the frequency characterization resulting from the last impact or an average of the frequency characterizations respectively resulting from the N last impacts, N being a number between two and four, with the frequency characterization resulting from the second last impact or with an average of the frequency characterizations respectively resulting from the M impacts just preceding the last impact, M being a number between two and four.

11. System according to any one of the claims 1 to 9, wherein at each of the successive impacts, the analysis unit (22; 122; 222) is configured to compare the frequency characterization resulting from the last impact or an average of the frequency characterizations respectively resulting from the N last impacts, N being a number between two and four, with a threshold.

12. Surgical kit, comprising:
- a prosthetic component (1; 101) to be assembled with a bone (P ; H) by impaction,
- an impact application device (10; 110) for applying successive impacts to the prosthetic component during assembly of the prosthetic component and the bone, the impact application device including a hand hammer (12; 112) and an impactor (11; 111) intended to transmit force from the hammer to the prosthetic component, and
- a system (20; 120) for monitoring use of the implant application device to assemble the prosthetic component with the bone by impaction, the system being according to any one of the preceding claims.

13. Surgical kit according to claim 12, wherein the prosthetic component is:
- an acetabular cup (1) of a hip prosthesis, intended to be assembled into an acetabulum (A) of a human pelvis (P) without cement, or
- a humeral stem (101) of a shoulder prosthesis, intended to be assembled into a medullary canal (C) of a human humerus (H) without cement, or
- a glenoid plate of a shoulder prosthesis, intended to be assembled into a glenoid cavity of a human scapula without cement.

14. Surgical kit, comprising:
- a first prosthetic component (102; 103) and a second prosthetic component (101), which are to be assembled together by impaction,
- an impact application device (210) for applying successive impacts to the first prosthetic component during assembly of the first prosthetic component and the second prosthetic component, the impact application device including a hand hammer (212) and an impactor (211) intended to transmit force from the hammer to the first prosthetic component, and
- a system (220) for monitoring use of the implant application device to assemble the first prosthetic component with the second prosthetic component by impaction, the system being according to any one of the claims 1 to 11.

15. Method for monitoring use of an impact application device (10; 110; 210) operated by a user to assemble by impaction a first prosthetic component (102; 103) with a second prosthetic component (101) held in an impaction table (T), the impact application device including a hand hammer (12; 112; 212) and an impactor (11; 111; 211) intended to transmit force from the hammer to the first prosthetic component, the method comprising:
- each time the impactor (11; 111; 211) is hit by the hammer (12; 112; 212) manipulated by hand by the user and thus applies an impact to the first prosthetic component during assembly of the first prosthetic component and the second prosthetic component, producing data representing acoustic vibrations (V_{A}) generated in the air and/or material vibrations (V_{M}) generated in the impact application device, from at least one vibrational sensor (21; 121; 221),
- calculating a frequency characterization of the vibrations for each impact applied by the impact application device to the first prosthetic component, from the corresponding data produced by the at least one vibrational sensor,
- comparing the frequency characterizations that are respectively calculated for successive impacts so as to provide at each of the successive impacts a first indication when the assembly between the first prosthetic component and the second prosthetic component is not fully seated then a second indication when the assembly between the first prosthetic component and the second prosthetic component is fully seated, and
- providing the user with feedback based on the first and second indications.

## Patentansprüche

1. System (20; 120; 220) zur Überwachung der Verwendung einer Schlagausübungsvorrichtung (10; 110; 210), die von einem Anwender betätigt wird, um eine prothetische Komponente (1; 101; 102; 103) durch Schlagbearbeitung mit einem Trägerelement (P; H; 101) zusammenzusetzen, wobei die Schlagausübungsvorrichtung einen von Hand geführten Hammer (12; 112; 212) und einen Schlagkörper (11; 111; 211), der dafür gedacht ist, eine Kraft von dem Hammer auf die prothetische Komponente zu übertragen, einschließt, wobei das System umfasst:
mindestens einen Schwingungssensor (21; 121; 221), der dazu dient, jedes Mal, wenn der Schlagkörper (11; 111; 211) von dem vom Anwender von Hand geführten Hammer (12; 112; 212) getroffen wird und somit einen Schlag auf die prothetische Komponente ausübt, während die prothetische Komponente und das Trägerelement zusammengesetzt werden, Daten zu produzieren, wobei die produzierten Daten in der Luft erzeugte akustische Schwingungen (V_{A}) und/oder in der Schlagausübungsvorrichtung erzeugte Materialschwingungen (V_{M}) darstellen,
eine Analyseeinheit (22; 122; 222), die dafür ausgelegt ist, sowohl eine Frequenzcharakteristik der Schwingungen für jeden von der Schlagausübungsvorrichtung auf die prothetische Komponente ausgeübten Schlag aus den entsprechenden, von dem mindestens einen Schwingungssensor produzierten Daten zu berechnen, als auch die Frequenzcharakteristiken, die jeweils für aufeinanderfolgende Schläge berechnet werden, zu vergleichen, um bei jedem von den aufeinanderfolgenden Schlägen entweder einen ersten Hinweis, wenn die prothetische Komponente und das Trägerelement noch nicht vollständig zusammengesetzt sind, oder einen zweiten Hinweis, wenn die prothetische Komponente und das Trägerelement vollständig zusammengesetzt sind, bereitzustellen, und
eine Anwenderschnittstelle (23; 123; 223), die dem Anwender Rückmeldungen auf Basis des ersten und des zweiten Hinweises bereitstellt.

2. System nach Anspruch 1, wobei der mindestens eine Vibrationssensor ein Mikrofon (21) aufweist, das dazu dient, Daten zu produzieren, welche die in der Luft erzeugten akustischen Schwingungen (V_{A}) darstellen, wobei das Mikrofon von der prothetischen Komponente (1) getrennt vorliegt.

3. System nach Anspruch 2, wobei das Mikrofon (21) auch von der Schlagausübungsvorrichtung (10) getrennt vorliegt.

4. System nach Anspruch 3, wobei das Mikrofon (21), die Analyseeinheit (22) und die Anwenderschnittstelle (23) in einer tragbaren Vorrichtung (40), wie etwa einem Laptop, einem Smartphone oder einem Pad, integriert sind.

5. System nach Anspruch 2, wobei das Mikrofon (21) in die Schlagausübungsvorrichtung (10) integriert ist.

6. System nach einem der vorangehenden Ansprüche, wobei der mindestens eine Schwingungssensor einen piezoelektrischen Sensor (121; 221) einschließt, der dazu dient, Daten, welche die in der Schlagausübungsvorrichtung (110; 210) erzeugten Materialschwingungen (V_{M}) darstellen, zu produzieren, wobei der piezoelektrische Sensor in die Schlagausübungsvorrichtung integriert ist.

7. System nach Anspruch 5 oder Anspruch 6, wobei die Analyseeinheit (22; 122; 222) und die Anwenderschnittstelle (23; 123; 223) auch in die Schlagausübungsvorrichtung (10; 110; 210) integriert sind.

8. System nach einem der vorangehenden Ansprüche, wobei die Frequenzcharakteristik, die von der Analyseeinheit (22) berechnet wird, eine natürliche Frequenz (NF1 - NF19) der akustischen Schwingungen (V_{A}) ist, die in der Luft erzeugt werden.

9. System nach einem der vorangehenden Ansprüche, wobei die Frequenzcharakteristik, die von der Analyseeinheit (122; 222) berechnet wird, eine Wellenfrequenz der in der Schlagausübungsvorrichtung (110; 210) erzeugten Materialschwingungen (V_{M}) ist.

10. System nach einem der vorangehenden Ansprüche, wobei die Analyseeinheit (22; 122; 222) dafür ausgelegt ist, bei jedem der aufeinanderfolgenden Schläge die Frequenzcharakteristik, die ein Ergebnis des letzten Schlages ist, oder einen Durchschnitt der Frequenzcharakteristiken, die jeweils ein Ergebnis der N letzten Schläge sind, wobei N eine Zahl zwischen zwei und vier ist, mit der Frequenzcharakteristik, die ein Ergebnis des vorletzten Schlages ist, oder einem Durchschnitt der Frequenzcharakteristiken, die jeweils ein Ergebnis der M Schläge sind, die dem letzten Schlag unmittelbar vorausgegangen sind, wobei M eine Zahl zwischen zwei und vier ist, zu vergleichen.

11. System nach einem der Ansprüche 1 bis 9, wobei die Analyseeinheit (22; 122; 222) dafür ausgelegt ist, bei jedem von den aufeinanderfolgenden Schlägen die Frequenzcharakteristik, die ein Ergebnis des letzten Schlages ist, oder einen Durchschnitt der Frequenzcharakteristiken, die jeweils ein Ergebnis der N letzten Schläge sind, wobei N eine Zahl zwischen zwei und vier ist, mit einem Schwellenwert zu vergleichen.

12. Chirurgisches Kit, umfassend:
eine prothetische Komponente (1; 101), die durch Schlagbearbeitung mit einem Knochen (P; H) zusammenzusetzen ist,
eine Schlagausübungsvorrichtung (10; 110) zur Ausübung aufeinanderfolgender Schläge auf die prothetische Komponente während des Zusammensetzens der prothetischen Komponente und des Knochens, wobei die Schlagausübungsvorrichtung einen von Hand zu führenden Hammer (12; 112) und einen Schlagkörper (11; 111), der dazu gedacht ist, die Kraft vom Hammer auf die prothetische Komponente zu übertragen, einschließt, und
ein System (20; 120) zur Überwachung des Gebrauchs der Schlagausübungsvorrichtung zum Zusammensetzen der prothetischen Komponente und des Knochens durch Schlagbearbeitung, wobei das System einem der vorangehenden Ansprüche entspricht.

13. Chirurgisches Kit nach Anspruch 12, wobei die prothetische Komponente ist:
eine Hüftgelenksschale (1) einer Hüftprothese, die dazu gedacht ist, ohne Zement in eine Hüftgelenkspfanne (A) eines menschlichen Beckens (P) eingesetzt zu werden, oder
ein Humerusschaft (101) einer Schulterprothese, der dazu gedacht ist, ohne Zement in einen Markkanal (C) eines menschlichen Oberarmknochen (H) eingesetzt zu werden, oder
eine Schulterplatte einer Schulterprothese, die dazu gedacht ist, ohne Zement in eine Schulterpfanne eines menschlichen Schulterblatts eingesetzt zu werden.

14. Chirurgisches Kit, umfassend:
eine erste prothetische Komponente (102; 103) und eine zweite prothetische Komponente (101), die durch Schlagbearbeitung zusammenzusetzen sind,
eine Schlagausübungsvorrichtung (210) zur Ausübung aufeinanderfolgender Schläge auf die erste prothetische Komponente während des Zusammensetzens der ersten prothetischen Komponente und der zweiten prothetischen Komponente, wobei die Schlagausübungsvorrichtung einen von Hand zu führenden Hammer (212) und einen Schlagkörper (211), der dazu gedacht ist, die Kraft vom Hammer auf die erste prothetische Komponente zu übertragen, einschließt, und
ein System (220) zur Überwachung des Gebrauchs der Schlagausübungsvorrichtung zum Zusammensetzen der ersten prothetischen Komponente und der zweiten prothetischen Komponente durch Schlagbearbeitung, wobei das System einem der vorangehenden Ansprüche 1 bis 11 entspricht.

15. Verfahren zum Überwachen der Verwendung einer Schlagausübungsvorrichtung (10; 110; 210), die von einem Anwender betätigt wird, um eine erste prothetische Komponente (102; 103) mit einer zweiten, auf einem Schlagbearbeitungstisch (T) gehaltenen prothetischen Komponente (101) durch Schlagbearbeitung zusammenzusetzen, wobei die Schlagausübungsvorrichtung einen von Hand zu führenden Hammer (12; 112; 212) und einen Schlagkörper (11; 111; 211), der dafür gedacht ist, eine Kraft von dem Hammer auf die prothetische Komponente zu übertragen, einschließt, wobei das Verfahren umfasst:
Produzieren von Daten, die in der Luft erzeugte akustische Schwingungen (V_{A}) und/oder in der Schlagausübungsvorrichtung erzeugte Materialschwingungen (V_{M}) aus mindestens einem Schwingungssensor (21; 121; 221) darstellen, jedes Mal, wenn der Schlagkörper (11; 111; 211) von dem vom Anwender von Hand geführten Hammer (12; 112; 212) getroffen wird und somit einen Schlag auf die erste prothetische Komponente ausübt, während die erste prothetische Komponente und die zweite prothetische Komponente zusammengesetzt werden,
Berechnen einer Frequenzcharakteristik der Schwingungen für jeden Schlag, der durch die Schlagausübungsvorrichtung auf die erste prothetische Komponente ausgeübt wird, aus den entsprechenden Daten, die von dem mindestens einen Schwingungssensor produziert werden,
Vergleichen der Frequenzcharakteristiken, die jeweils für aufeinanderfolgende Schläge berechnet werden, um bei jedem der aufeinanderfolgenden Schläge einen ersten Hinweis, wenn das Zusammensetzen zwischen der ersten prothetischen Komponente und der zweiten prothetischen Komponente nicht vollständig ist, und dann einen zweiten Hinweis, wenn das Zusammensetzen zwischen der ersten prothetischen Komponente und der zweiten prothetischen Komponente vollständig ist, bereitzustellen, und
Bereitstellen einer Rückmeldung für den Anwender auf Basis der ersten und zweiten Hinweise.

## Revendications

1. Système (20 ; 120 ; 220) destiné à surveiller l'utilisation d'un dispositif d'impact (10 ; 110 ; 210) actionné par un utilisateur afin d'assembler un composant de prothèse (1 ; 101 ; 102 ; 103) avec un élément de support (P ; H ; 101) par impaction, le dispositif d'impact comprenant un marteau à main (12 ; 112 ; 212) et un impacteur (11 ; 111 ; 211) destiné à transmettre une force entre le marteau et le composant de prothèse, le système comprenant :
- au moins un capteur de vibrations (21 ; 121 ; 221) capable de produire des données à chaque fois que l'impacteur (11 ; 111 ; 211) est heurté par le marteau (12 ; 112 ; 212) manipulé à la main par l'utilisateur, et qui applique ainsi un impact sur le composant de prothèse pendant l'assemblage du composant de prothèse et de l'élément de support, les données produites représentant les vibrations acoustiques (V_{A}) générées dans l'air et/ou les vibrations matérielles (V_{M}) générées dans le dispositif d'impact,
- une unité d'analyse (22 ; 122 ; 222) qui est configurée pour calculer une caractérisation de fréquence des vibrations pour chaque impact appliqué par le dispositif d'impact au composant de prothèse, à partir des données correspondantes produites par le au moins un capteur de vibrations, et pour comparer les caractérisations de fréquence qui sont respectivement calculées pour des impacts successifs de façon à fournir, à chacun des impacts successifs, soit une première indication lorsque l'assemblage entre le composant de prothèse et l'élément de support n'est pas entièrement en place, soit une seconde indication lorsque l'assemblage entre le composant de prothèse et l'élément de support est entièrement en place,
et
- une interface utilisateur (23 ; 123 ; 223) qui fournit un retour à l'utilisateur sur la base des première et seconde indications.

2. Système selon la revendication 1, dans lequel le au moins un capteur de vibrations comprend un microphone (21) capable de produire des données qui représentent les vibrations acoustiques (V_{A}) générées dans l'air, le microphone étant distinct du composant de prothèse (1).

3. Système selon la revendication 2, dans lequel le microphone (21) est également distinct du dispositif d'impact (10).

4. Système selon la revendication 3, dans lequel le microphone (21), l'unité d'analyse (22) et l'interface utilisateur (23) sont intégrés à un dispositif portatif (40), tel qu'un ordinateur portable, un mobile multifonction ou une tablette.

5. Système selon la revendication 2, dans lequel le microphone (21) est intégré au dispositif d'impact (10).

6. Système selon l'une quelconque des revendications précédentes, dans lequel le au moins un capteur de vibrations comprend un capteur piézoélectrique (121 ; 221) capable de produire des données qui représentent les vibrations matérielles (V_{M}) générées dans le dispositif d'impact (110 ; 210), le capteur piézoélectrique étant intégré au dispositif d'impact.

7. Système selon la revendication 5 ou 6, dans lequel l'unité d'analyse (22 ; 122 ; 222) et l'interface utilisateur (23 ; 123 ; 223) sont également intégrées au dispositif d'impact (10 ; 110 ; 210).

8. Système selon l'une quelconque des revendications précédentes, dans lequel la caractérisation de fréquence calculée par l'unité d'analyse (22) est une fréquence naturelle (NF1 - NF19) des vibrations acoustiques (V_{A}) générées dans l'air.

9. Système selon l'une quelconque des revendications précédentes, dans lequel la caractérisation de fréquence calculée par l'unité d'analyse (122 ; 222) est une fréquence d'onde des vibrations matérielles (V_{M}) générées dans le dispositif d'impact (110 ; 210).

10. Système selon l'une quelconque des revendications précédentes, dans lequel, à chacun des impacts successifs, l'unité d'analyse (22 ; 122 ; 222) est configurée pour comparer la caractérisation de fréquence qui résulte du dernier impact ou une moyenne des caractérisations de fréquences qui résultent respectivement des N derniers impacts, N étant un nombre compris entre deux et quatre, avec la caractérisation de fréquence qui résulte de l'avant-dernier impact ou avec une moyenne des caractérisations de fréquence qui résultent respectivement des M impacts qui ont précédé le dernier impact, M étant un chiffre compris entre deux et quatre.

11. Système selon l'une quelconque des revendications 1 à 9, dans lequel, à chacun des impacts successifs, l'unité d'analyse (22 ; 122 ; 222) est configurée pour comparer la caractérisation de fréquence qui résulte du dernier impact ou une moyenne des caractérisations de fréquence qui résultent respectivement des N derniers impacts, N étant un nombre compris entre deux et quatre, avec un seuil.

12. Trousse chirurgicale, comprenant :
- un composant de prothèse (1 ; 101) à assembler avec un os (P ; H) par impaction,
- un dispositif d'impact (10 ; 110) destiné à appliquer des impacts successifs au composant de prothèse pendant l'assemblage du composant de prothèse et de l'os, le dispositif d'impact comprenant un marteau à main (12 ; 112) et un impacteur (11 ; 111) destiné à transmettre une force entre le marteau et le composant de prothèse, et
- un système (20 ; 120) destiné à surveiller l'utilisation du dispositif d'impact afin d'assembler le composant de prothèse avec l'os par impaction, le système étant selon l'une quelconque des revendications précédentes.

13. Trousse chirurgicale selon la revendication 12, dans laquelle le composant de prothèse est :
- un cotyle (1) d'une prothèse de hanche, destiné à être assemblé dans un acétabulum (A) d'un pelvis humain (P) sans ciment, ou
- une tige humérale (101) d'une prothèse d'épaule, destinée à être assemblée dans un canal médullaire (C) d'un humérus humain (H) sans ciment, ou
- une plaque glénoïde d'une prothèse d'épaule, destinée à être assemblée dans une cavité glénoïde d'une omoplate humaine sans ciment.

14. Trousse chirurgicale, comprenant :
- un premier composant de prothèse (102 ; 103) et un second composant de prothèse (101), qui doivent être assemblés ensemble par impaction,
- un dispositif d'impact (210) destiné à appliquer des impacts successifs au premier composant de prothèse pendant l'assemblage du premier composant de prothèse et du second composant de prothèse, le dispositif d'impact comprenant un marteau à main (212) et un impacteur (211) destiné à transmettre une force entre le marteau et le premier composant de prothèse, et
- un système (220) destiné à surveiller l'utilisation du dispositif d'impact afin d'assembler le premier composant de prothèse avec le second composant de prothèse par impaction, le système étant selon l'une quelconque des revendications 1 à 11.

15. Procédé de surveillance de l'utilisation d'un dispositif d'impact (10 ; 110 ; 220) actionné par un utilisateur afin d'assembler par impaction un premier composant de prothèse (102 ; 103) avec un second composant de prothèse (101) maintenu dans une table d'impaction (T), le dispositif d'impact comprenant un marteau à main (12 ; 112 ; 212) et un impacteur (11 ; 111 ; 211) destiné à transmettre une force entre le marteau et le premier composant de prothèse, le procédé comprenant :
- à chaque fois que l'impacteur (11 ; 111 ; 211) est heurté par le marteau (12 ; 112 ; 212) manipulé à la main par l'utilisateur et applique ainsi un impact au premier composant de prothèse pendant l'assemblage du premier composant de prothèse et du second composant de prothèse, la production de données qui représentent les vibrations acoustiques (V_{A}) générées dans l'air et/ou les vibrations matérielles (V_{M}) générées dans le dispositif d'impact, à partir d'au moins un capteur de vibrations (21 ; 121 ; 221),
- le calcul d'une caractérisation de fréquence des vibrations pour chaque impact appliqué par le dispositif d'impact au premier composant de prothèse, à partir des données correspondantes produites par le au moins un capteur de vibrations,
- la comparaison des caractérisations de fréquence qui sont respectivement calculées pour des impacts successifs de façon à fournir, à chacun des impacts successifs, une première indication lorsque l'assemblage entre le premier composant de prothèse et le second composant de prothèse n'est pas entièrement en place, puis une seconde indication lorsque l'assemblage entre le premier composant de prothèse et le second composant de prothèse est entièrement en place, et
- la fourniture d'un retour à l'utilisateur sur la base des première et seconde indications.
